# EUROPÄISCHE PATENTSCHRIFT

(11) **EP 3 179 931 B1**
(45) Veröffentlichungstag und Bekanntmachung des Hinweises auf die Patenterteilung: **25.04.2018**
(21) Anmeldenummer: 15749722.3
(22) Anmeldetag: 05.08.2015
(51) Int. Cl.: A61B 17/29, A61B 17/00, A61B 18/14

(54) **MEDIZINISCHES INSTRUMENT FÜR ENDOSKOPE CHIRURGIE**
MEDICAL INSTRUMENT FOR ENDOSCOPIC SURGERY
INSTRUMENT MEDICAL POUR LA CHIRURGIE ENDOSCOPIQUE

(30) Priorität: 15.08.2014 DE 102014012036
(43) Veröffentlichungstag der Anmeldung: 21.06.2017
(73) Patentinhaber: Olympus Winter & Ibe GmbH, 22045 Hamburg (DE)
(72) Erfinder: AUE, Thomas, 22880 Wedel (DE)
(74) Vertreter: Meissner Bolte Partnerschaft mbB
(86) Internationale Anmeldenummer: PCT/EP2015/001611
(87) Internationale Veröffentlichungsnummer: WO 2016/023624

(56) Entgegenhaltungen:
- DE-A1- 10 038 085
- DE-A1-102006 038 517
- DE-A1-102006 062 421
- DE-A1-102011 007 121
- DE-C1- 10 064 623
- DE-C1- 19 853 305
- US-A- 5 741 286

## Beschreibung

Die Erfindung betrifft ein medizinisches Instrument für endoskope Chirurgie gemäß dem Oberbegriff des Anspruchs 1.
Instrumente für endoskope Chirurgie umfassen in der Regel ein Schaftrohr mit einem im proximalen Endbereich des Schaftrohres angeordneten Verbindungskörper, eine durch das Schaftrohr geführte Zug-/Druckstange mit einem im distalen Endbereich der Stange befestigten Endeffektor und einen mit der Stange koppelbaren Handgriff zur Führung des Instruments und zur Betätigung des Endeffektors. Zu Reinigungszwecken und für die Anpassbarkeit an unterschiedliche Einsatzzwecke sind solche Instrumente üblicherweise zerlegbar ausgeführt.
Zur Herstellung einer lösbaren Verbindung zwischen dem Handgriff und dem Schaftrohr, ist der Handgriff üblicherweise mit einem Kuppelstück an den Verbindungskörper ankuppelbar. Der Verbindungskörper dient auch als Drehgriff zur Verdrehung eines an dem distalen Ende der Stange angeordneten Endeffektors. Dafür ist die Stange mit dem Endeffektor typischerweise drehgesichert mit dem Schaftrohr verbindbar. Das proximale Ende der durch das Schaftrohr geführten Stange ist bei solchen Ausführungen formschlüssig mit dem Handgriff kuppelbar.
Ein wichtiger Aspekt bei der Konstruktion zerlegbarer Instrumente der eingangs genannten Art ist der Mechanismus zur Herstellung einer Kupplungsverbindung zwischen den Einzelzeilen. Die Kuppelmechanismen sind üblicherweise so ausgeführt, dass zumindest die Entkupplung von zwei Teilen durch Überwindung eines federvorgespannten Verriegelungsmechanismus durchgeführt wird. Häufig wird dazu ein federnd in einem Querschacht des Verbindungskörpers gelagerter Sperrschieber verwendet, der über Fingerdruck betätigbar ist. Bei der Ausführung des Sperrschiebers wird besonderes Augenmerk darauf gelegt, einerseits eine schnelle und einfache Bedienbarkeit zu unterstützen und andererseits eine hohe Sicherheit gegen Fehlfunktionen zu gewährleisten. Zur Herstellung schmaler, leichter Instrumente ist darüberhinaus eine kompakte Integration des Sperrschiebers in den Verbindungskörper gewünscht.

Aus DE 10064623 C1 ist ein gattungsgemäßes Instrument gemäß des Oberbegriffs von Anspruch 1 bekannt, das mehrteilig auseinandernehmbar ausgebildet ist. Gemäß diesem Stand der Technik kann vorgesehen sein, einen in einem Schacht eines Verbindungskörpers gelagerten Sperrschieber mit Federkraft in einer Haltestellung zu halten. Dafür wird vorgeschlagen, eine Schraubenfeder außerhalb des Verbindungskörpers koaxial zu dem Sperrschieber anzuordnen, wobei sich ein erstes Ende der Schraubenfeder im Randbereich der Schachtöffnung an einer Wandung des Verbindungskörpers abstützt und das zweite Ende der Schraubenfeder gegen einen radialen Vorsprung des Sperrschiebers drückt.
Eine derartige Konstruktion mit einer an der Außenseite des medizinischen Instrumentes exponierten Feder hat sich in verschiedener Hinsicht als nachteilig gezeigt. Ein Aspekt ist die schlechte Reinigbarkeit solcher Instrumente. Außen an dem Gerät angeordnete Federelemente bilden diverse Kanten und Spalten, die nur aufwändig von Keimen und Rückständen von mit dem Instrument in Verbindung kommenden Substanzen reinigbar sind. Außerdem besteht die Gefahr, dass an dem Federelement vorbeistreifende Gewebeteile oder andere Materialien wie zum Beispiel Fäden zum Verschließen von Gewebeöffnungen an den Kanten des Federelementes hängen bleiben. Dies kann zu Verklemmungen des Haltemechanismus beziehungsweise zu Behinderungen beim Einsatz des Instrumentes führen.
Aus DE 102011007121 A1 ist eine Handhabungseinrichtung für ein mikroinvasivchirurgisches Instrument bekannt mit einer Schaftkupplung zum lösbaren mechanischen Koppeln der Handhabungseinrichtung mit einem proximalen Ende eines Schaftes, wobei die Schaftkupplung ausgebildet ist, ein in die Schaftkupplung eingesetztes proximales Ende des Schaftes in einer vorbestimmten Position zu arretieren. Die Arretierung wird durch Betätigung eines federgelagerten Entriegelungsknopfes gelöst.

Aufgabe der vorliegenden Erfindung ist es, ein medizinisches Instrument der eingangs genannten Art bereitzustellen, das eine gute Bedienbarkeit und Reinigbarkeit aufweist und zugleich einen verbesserten Schutz gegen Fehlfunktionen bietet.

Diese Aufgabe wird gelöst durch einen Gegenstand mit den Merkmalen des Patentanspruchs 1.

Erfindungsgemäß ist ein medizinisches Instrument für endoskopische Chirurgie, aufweisend ein Schaftrohr mit einem distalen und einem proximalen Ende und einen im Bereich des proximalen Endes des Schaftrohres angeordneten Verbindungskörper, wobei das Schaftrohr und der Verbindungskörper zur Durchführung eines langgestreckten Betätigungselements axial von einem gemeinsamen Kanal durchsetzt sind, einen an den Verbindungskörper ankuppelbaren Endkörper mit einem daran befestigten Handgriff zur Bedienung des medizinischen Instrumentes, einen Sperrschieber, der in einem im Winkel zu dem Kanal ausgerichteten Schacht innerhalb des Verbindungskörpers verschiebbar gelagert ist, wobei der Sperrschieber mit einem Betätigungsbereich aus dem Verbindungskörper herausragt und per Fingerdruck auf den Betätigungsbereich in eine Verriegelungs- und eine Freigabestellung betätigbar ist, derart, dass der an den Verbindungskörper angekuppelte Endkörper in der Verriegelungsstellung formschlüssig an dem Verbindungskörper verriegelt ist und in der Freigabestellung von dem Verbindungskörper abnehmbar ist, und wobei der Sperrschieber mit Federkraft eines Federelements in der Verriegelungsstellung gehalten ist und wobei erfindungsgemäß vorgesehen ist, dass das Federelement in dem Schacht zwischen dem Betätigungsbereich und dem Kanal angeordnet ist.

Bei einer unterhalb des Kanals angeordneten Federelements müssen verhältnismäßig starke Materialschichten in umliegenden Wandungsbereichen des Verbindungskörpers vorgesehen sein, was zu einem insgesamt breiteren und schweren Verbindungskörper und damit zu Handhabungsnachteilen des Instruments führt. Eine derartige Konstruktion ist aus DE 10 2006 038 517 A1 bekannt. Bei der Platzierung eines zum Beispiel als Schraubenfeder ausgebildeten Federelements unterhalb des Sperrschiebers ist nämlich typischerweise die Einarbeitung einer planen Auflagefläche in die Innenwandung des Verbindungskörpers notwendig. Dies setzt bei einer außen verrundeten Geometrie des Verbindungskörpers eine entsprechende Wanddicke voraus. Zudem müsste unterhalb des Sperrschiebers ein Leervolumen vorgesehen sein, das groß genug ist, das Federelement aufzunehmen und einen ausreichenden Federweg erlaubt.

Die Platzierung des Federelements zwischen dem Betätigungsbereich des Sperrschiebers und dem insbesondere axial durch den Verbindungskörper verlaufenden Kanal bietet die Möglichkeit, den Raum innerhalb des Verbindungskörpers optimal auszunutzen. In dem Schacht unterhalb des Sperrschiebers ist bei dieser Ausgestaltung nur noch wenig freies Volumen notwendig, und zwar gerade so viel, dass ein gewünschter Verschiebungsweg des Sperrschiebers zugelassen ist. Der Raum unterhalb des Sperrschiebers kann bei der Verschiebung vollständig ausgenutzt werden.

Grundsätzlich ist daran gedacht, dass der Sperrschieberschacht winklig zum Kanal, also insbesondere schräg zur Längsachse des Verbindungskörpers verläuft. Damit ist die Ausgestaltung des Verriegelungsmechanismus zur Verriegelung des den Handgriff tragenden Endkörpers vereinfacht. An dem Sperrschieber kann nämlich in einer einfachen Ausführung ein Haken angeordnet sein, der der Bewegung des Sperrschiebers zwangsgeführt folgt und zur Verriegelung in eine Ringnut des Endkörpers eingreift. Bevorzugt ist daran gedacht, dass der Schacht zur Lagerung des Sperrschiebers quer zur Längsachse des Schaftrohres ausgeführt ist.

Für eine gute Bedienbarkeit des Verriegelungsmechanismus ist vorgesehen, dass der Sperrschieber mit dem Betätigungsbereich aus einer Öffnung des Verbindungskörpers herausragt. Vorzugsweise ist der Betätigungsbereich integral mit dem Sperrschieber ausgebildet. Alternativ ist denkbar, dass der Betätigungsbereich abnehmbar an dem Sperrschieber befestigt ist.

In einer erfindungsgemäßen Ausgestaltung ist daran gedacht, dass sich das Federelement mit einem in Federrichtung ersten, dem Betätigungsbereich abgewandten Ende an einer im Bereich des Kanals angeordneten Stützfläche abstützt und sich mit einem in Federrichtung zweiten, dem Betätigungsbereich zugewandten Ende an einer Stoßfläche des Sperrschiebers abstützt.

Abhängig von der Form des Federelements ist denkbar, dass das Federelement über Abstützelemente, wie z.B. Scheiben, Stege oder dergleichen, an der Stoßfläche des Sperrschiebers unterhalb des Betätigungsbereiches und/oder an der Stützfläche im

Bereich des Kanals abgestützt ist. Die Stützfläche kann zum Beispiel eine in der Schachtwandung eingelassene Ringnut sein. Analog dazu kann die Stoßfläche eine Ringnut am Umfang des Sperrschiebers sein. Alternativ kann die Stützfläche an einem separat ausgeführten Bauteil des Verbindungskörpers vorgesehen sein. Zum Beispiel kann die Stützfläche an einer axial in den Verbindungskörper einsteckbaren Hülse vorgesehen sein. Ein derartiges Bauteil kann zum Beispiel ein Führungselement zur Führung des an den Verbindungskörper ankuppelbaren Endkörper sein. Bei einem hülsenartigen Bauteil ist daran gedacht, dass der axiale Durchgang der Hülse den Kanal für das Betätigungselement umfasst. Die Stützfläche ist dabei an einer Außenwandung der Hülse vorgesehen. Das hülsenartige Bauteil mit der Stützfläche kann auch zur Durchleitung einer in das Instrument eingekoppelten elektrischen Spannung ausgeführt sein. Dafür kann das Bauteil zum Beispiel aus einem elektrisch leitfähigen Material hergestellt sein.

In einer vorteilhaften Ausgestaltung ist daran gedacht, dass das Federelement in einer Durchbrechung des Sperrschiebers angeordnet ist. Vorzugsweise ist der Sperrschieber mit der Durchbrechung tunnelartig durchsetzt, insbesondere quer zur Schachtrichtung. Besonders bevorzugt ist die Durchbrechung quer zur Kanalrichtung in den Sperrschieber eingearbeitet. Die Durchbrechung kann in axiale Richtung des Verbindungskörpers also distal und proximal von Wandflächen des Sperrschiebers begrenzt sein.

Die Integration des Federelements in eine Durchbrechung des Sperrschiebers bietet gleich zwei Vorteile. Einerseits werden die eingangs genannten Nachteile einer verschlechterten Reinigbarkeit beziehungsweise der Bildung von zusätzlichen Kanten und Spalten an der Außenseite des Verbindungskörpers vermieden und andererseits wird der Platz innerhalb des Verbindungskörpers optimal ausgenutzt.

Bei einer speziellen Variante des medizinischen Instrumentes ist vorgesehen, dass an dem Endkörper eine Anschlussstelle angeordnet ist, zur Einkupplung einer elektrischen Spannung in das Schaftrohr und/oder in ein durch das Schaftrohr geführtes Betätigungselement. Wie erwähnt, kann das Betätigungselement eine durch den Kanal geführte Zug-/Druckstange sein.

Als elektrische Spannung kann z.B. ein Hochfrequenzsignal eines Hochfrequenzgenerators und/oder eine Gleichspannung bzw. allgemein eine Wechselspannung vorgesehen sein. Desweiteren kann vorgesehen sein, dass das medizinische Instrument monopolar oder bipolar ausgeführt ist. Das heißt, an der Anschlussstelle, die vorzugsweise als Steckkontakt ausgebildet ist, können ein oder zwei Anschlusskontakte für elektrische Spannungssignale vorgesehen sein.

Bei einem in den Verbindungskörper eingekuppelten Endkörper ist daran gedacht, dass mindestens eine elektrische Spannung über einen elektrisch leitenden Pfad des Endkörpers an mindestens ein elektrisch leitendes Bauteil innerhalb des Verbindungskörpers weitergeleitet wird. Es ist weiter daran gedacht, dass eine elektrische Spannung über das elektrisch leitende Bauteil innerhalb des Verbindungskörpers im Bereich des Sperrschieberschachtes an einen elektrisch leitenden Pfad des Schaftrohres weitergeleitet wird. Über den elektrisch leitenden Pfad des Schaftrohres kann die elektrische Spannung an einen im distalen Endbereich des Schaftrohres angeordneten Endeffektor übertragen werden. Es zudem daran gedacht, dass das elektrisch leitende Bauteil die erfindungsgemäße Stützfläche zur Abstützung der Feder aufweist. Dies kann die Bauteilanzahl bzw. die Bauteilkomplexität des Instruments verringert werden.

In einer bevorzugten Variante des Instrumentes ist vorgesehen, dass der Sperrschieber eine das Betätigungselement durchlassende Öffnung aufweist. Bevorzugt ist das Federelement oberhalb der Öffnung angeordnet. Die Anordnung des Federelements oberhalb der Öffnung und unterhalb des Betätigungsbereiches des Sperrschiebers führt zu einer verbesserten Raumausnutzung innerhalb des Sperrschieberschachtes, beziehungsweise innerhalb des Verbindungskörpers.

Die Öffnung des Sperrschiebers hat vorzugsweise eine Innenkontur, mit der eine Drehsicherung mit einem durch das Schaftrohr und durch den Verbindungskörper geführten Betätigungselement herstellbar ist. Dafür ist daran gedacht, dass die Öffnung einen Durchlassbereich und einen Fassbereich aufweist, wobei der Sperrschieber in der Freigabestellung einen proximalen Abschnitt eines durch den Kanal geführten Betätigungselements durch den Durchlassbereich frei durchlässt und in der Verriegelungsstellung einen Eingriffsbereich des Betätigungselements drehformschlüssig mit dem Fassbereich umgreift. Details der Drehsicherung können beispielsweise wie in der hiermit ausdrücklich in Bezug genommenen Druckschrift DE 100 64 623 C1 ausgeführt sein.

Erfindungsgemäß ist vorgesehen, dass das Federelement als konische Schraubenfeder ausgeführt ist. Weiter bevorzugt ist vorgesehen, dass sich die Querschnittsfläche des als Schraubenfeder ausgebildeten Federelements von dem ersten Ende zum zweiten Ende hin verjüngt. Insbesondere ist daran gedacht, dass eine Kegelstumpffeder verwendet wird. Insbesondere bei einer radialsymmetrischen Ausführung des Federelements ist daran gedacht, dass das Federelement koaxial zum Sperrschieber in dem Schacht angeordnet ist.

Die konische Ausführung der Schraubenfeder vereinfacht den konstruktiven Aufbau des Sperrschiebers. Das verjüngte Ende der Schraubenfeder, das insbesondere schmaler als der Querschnitt des Sperrschiebers ausgebildet ist, kann dafür in einfacher Weise an einer Stoßfläche unterhalb des Betätigungsbereichs des Sperrschiebers abgestützt sein. Das breite Ende der Schraubenfeder, das insbesondere seitlich über den Querschnitt des Sperrschiebers hinausragt, kann dafür auf einer Stützfläche im Bereich des Kanals abgestützt sein. Abstützmittel am unteren Ende und/oder am oberen Ende der Feder, wie z.B. Unterlegscheiben, Stege oder dergleichen, können bei dieser Ausgestaltung entfallen. Dies verringert die Bauteilkomplexität und die Fehleranfälligkeit des Kupplungsmechanismus.

Bei der Verwendung von monopolaren beziehungsweise bipolaren Instrumenten kann, wie erläutert, vorgesehen sein, eine elektrische Spannung über ein dafür ausgebildetes Bauteil innerhalb des Verbindungskörpers an das Schaftrohr des Instruments weiterzuleiten. Wie erwähnt, kann die Stützfläche zur Abstützung des Federelements innerhalb des Verbindungskörpers an dem die elektrische Spannung leitende Bauteil angeordnet sein. In einer einfachen Ausführung kann das Bauteil aus einem elektrischen leitfähigen Vollmaterial bestehen, zum Beispiel ein Metall wie Edelstahl oder dergleichen. Wenn das Federelement ebenfalls aus einem elektrisch leitfähigen Material wie z.B. Edelstahl besteht, ist die sich auf dem Bauteil abstützende Feder ebenfalls mit der elektrischen Spannung beaufschlagt.

Bei derartigen Konstruktionen ist die Einhaltung von Normen, betreffend die elektrische Sicherheit des Instruments zu berücksichtigen. Da das obere Ende der Schraubenfeder direkt unterhalb des Betätigungsbereiches des Sperrschiebers endet, wird der Abstand zwischen einem elektrisch leitfähigen Punkt innerhalb des Verbindungskörpers, insbesondere innerhalb des Sperrschieberschachtes, und der Außenseite des Verbindungskörpers verringert. Bei dieser Konstruktion kann die Gefahr bestehen, dass die elektrische Spannung auf einen Körperteil des Bedieners des medizinischen Instrumentes überschlägt bzw. weitergeleitet wird. Insbesondere besteht diese Gefahr beim Betätigen des Sperrschiebers mit einem Fingerdruck auf den Betätigungsbereich.

Das elektrische Signal kann insbesondere durch einen Spalt zwischen dem Sperrschieber und der Innenwandung des Sperrschieberschachtes auf einen Körperteil des Bedieners übertragen werden. Verstärkt wird diese Gefahr, wenn z.B. Flüssigkeiten beziehungsweise Feuchtigkeit in den Spalt zwischen Sperrschieber und Innenwandung des Sperrschieberschachtes eindringen.

Durch die konstruktive Ausführung der Schraubenfeder als Konus- bzw. Kegelstumpffeder wird die Gefahr eines Überspringens der elektrischen Spannung auf den Bediener verringert. Dabei ist daran gedacht, dass das obere, sich an einer Stoßfläche des Sperrschiebers unterhalb des Betätigungsbereiches abstützende Ende gegenüber dem unteren, sich an einer Stützfläche im Bereich des Kanals abstützenden Ende verjüngt ist.

Eine konisch ausgeführte Schraubenfeder verbessert sowohl in mechanischer als auch in elektrischer Sicht die Funktionssicherheit des Instruments.

In einer bevorzugten Ausgestaltung kann vorgesehen sein, dass der Sperrschieber zumindest in dem für das Federelement vorgesehenen Abschnitt zwischen dem Betätigungsbereich und dem Kanal einen Querschnitt aufweist, der in einer ersten Richtung schmaler und in einer zweiten Richtung breiter ist als ein Querschnitt am ersten Ende des Federelements. Diesbezüglich kann auch vorgesehen sein, dass das Federelement zumindest in dem dafür vorgesehenen Abschnitt des Sperrschiebers an seinem ersten Ende den Querschnitt des Sperrschiebers zumindest bereichsweise überragt.

Bevorzugt ist daran gedacht, dass der Sperrschieber einen im Wesentlichen rechteckigen Querschnitt aufweist. Besonders bevorzugt ist der Querschnitt des Sperrschiebers in axiale Richtung des Schaftrohres länger als quer dazu ausgebildet. Der Querschnitt des Sperrschiebers ist also bevorzugt länglich ausgebildet, wobei der Sperrschieber in seiner Gebrauchsstellung innerhalb des Schachtes mit seiner langen Seite bevorzugt quer zur Längsrichtung des Verbindungskörpers ausgerichtet ist. Dies hat konstruktive Vorteile für die Verriegelung des Betätigungselements an der axial orientierten Öffnung des Sperrschiebers.

Erfindungsgemäß kann vorgesehen sein, dass eine konisch geformte Schraubenfeder seitlich in die Durchbrechung des Sperrschiebers einsteckbar ist. Insbesondere ist vorgesehen, dass die Schraubenfeder von zwei gegenüberliegenden, die Durchbrechung begrenzenden Wandflächen des Sperrschiebers eingefasst ist und zumindest an ihrem ersten, unteren Ende quer zwischen den gegenüberliegenden Wänden des Sperrschiebers über den Rand der Durchbrechung hinausragt. Dabei kann sich die Feder mit den über den Rand der Durchbrechung ragenden Bereichen gegen ein in dem Verbindungskörper ortsfest zu dem Sperrschieber gelagertes Bauteil abstützten. Dieses Bauteil kann - wie erwähnt - ein elektrisch leitfähiges Bauteil sein. Die mit der Feder in Kontakt stehende Stützfläche kann mit einem elektrischen Signal bzw. einer elektrischen Spannung beaufschlagt sein.

Für eine gute Bedienbarkeit des medizinischen Instrumentes ist daran gedacht, dass der Endkörper in seiner an den Verbindungskörper angekuppelten und von dem Sperrschieber gehaltenen Gebrauchsstellung drehbar an dem Verbindungskörper gelagert ist. Dies ermöglicht eine Verdrehung des Handgriffs gegenüber dem Schaftrohr bzw. einem drehfest mit dem Schaftrohr und/oder dem Verbindungskörper verbundenen Endeffektors.

In den Zeichnungen sind Ausführungsbeispiele der Erfindung schematisch dargestellt. Es zeigen:
- Fig. 1: einen Achsschnitt durch ein erfindungsgemäßes Instrument,
- Fig. 2: eine schematische Ansicht des Sperrschiebers im Schnitt nach Linie 2 bis 2 in Fig. 1;
- Fig. 3: eine vergrößerte Darstellung eines Teilbereiches des Verbindungskörpers mit Sperrschieber aus Fig. 1, und
- Fig. 4: den Endkörper aus Fig. 1 im Schnitt mit einem daran befestigten Handgriff.

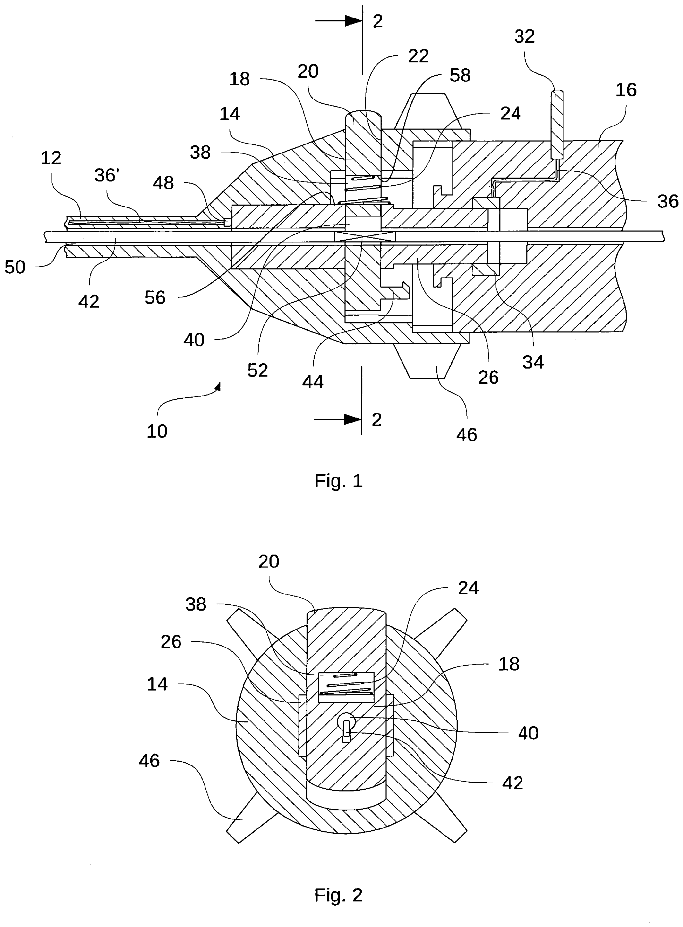

Fig. 1 zeigt in einer schematischen Schnittdarstellung von der Seite ein erfindungsgemäßes Instrument 10 mit einem langgestreckten Schaftrohr 12, an dem ein Verbindungskörper 14 befestigt ist. Der Verbindungskörper 14 hat einen Querschacht 22, in dem ein Sperrschieber 18 verschiebbar gelagert ist. Der Sperrschieber 18 hat an seinem aus dem Schacht 22 ragenden Ende einen Betätigungsbereich 20, mit dem der Sperrschieber 18 zum Beispiel per Fingerdruck gegen eine durch die Feder 24 aufgebrachte Federkraft in den Schacht 22 verschiebbar ist. An dem Sperrschieber 18 ist ein Riegelhaken 44 befestigt, mit dem ein in eine axiale Bohrung am proximalen Ende des Verbindungskörpers 14 einsteckbarer Endkörper 16 formschlüssig verriegelbar ist. Dafür greift der Riegelhaken 44 in eine Nut des Endkörpers 16 ein. Insbesondere kann die Nut des Endkörpers 16 als Ringnut ausgebildet sein, so dass der Endkörper 16 drehbar gegenüber dem Schaftrohr 12 an den Verbindungskörper 14 ankuppelbar ist. Vorzugsweise ist der Endkörper zumindest bereichsweise zylindrisch ausgeführt. Die Ringnut kann - wie dargestellt - ein radial in Richtung der Längsachse des Endkörpers nach innen ausgenommener Ringspalt sein. Der durch die Ringnut gebildete Ringspalt hat also bevorzugt eine radial nach außen gerichtete Spaltöffnung.

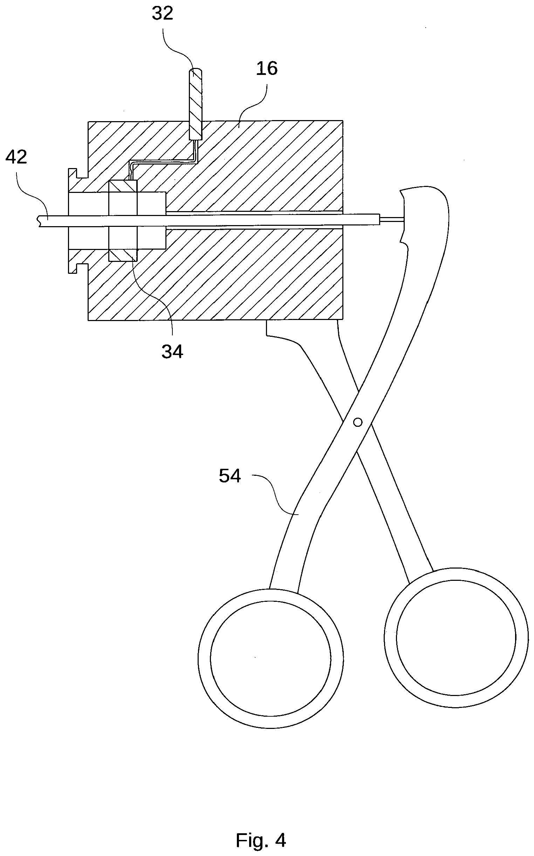

Erfindungsgemäß ist an dem Endkörper 16 ein Handgriff 54 befestigt (vgl. Fig. 4), mit dem das medizinische Instrument 10 bedienbar ist. Insbesondere kann vorgesehen sein, dass ein durch das Schaftrohr 12 geführtes Betätigungselement 42 durch eine Bohrung im Endkörper 16 ragt und mit seinem proximalen Ende mit dem Handgriff 54 kuppelbar (vgl. Fig. 4) ist, derart dass ein am distalen Ende des Schaftrohres 12 mit dem Betätigungselement 42 gekuppelter Endeffektor mittel des Handgriffs 54 betätigbar ist. Ein derartiger Endeffektor (nicht dargestellt) kann zum Beispiel eine Zange, eine Schere ein Messer oder dergleichen sein. Das Betätigungselement 42 kann als Zug-/Druckstange ausgebildet sein. Ein zur Durchführung des Betätigungselements 42 vorgesehener gemeinsamer axialer Kanal 50 durchsetzt das medizinische Instrument vom distalen Ende des Schaftrohres 12 bis zum proximalen Ende des Endkörpers 16.

Mit der Verstellung des Handgriffs 54 und dem damit bewegungsgekoppelten Betätigungselement 42 können zum Beispiel zwei Branchen eines Endeffektors für eine Öffnungs- bzw. Schließbewegung betätigbar sein. Details der Betätigungsmechanik und die konstruktive Ausgestaltung des Handgriffs und/oder eines am distalen Ende des Schaftrohres 12 angeordneten Endeffektors können wie in der hiermit ausdrücklich in Bezug genommenen DE 100 64 623 C1 und/oder der ebenfalls ausdrücklich in Bezug genommenen DE 198 53 305 C1 ausgestaltet sein.

Wie in Figur 1 erkennbar, ist das als Schraubenfeder ausgebildete Federelement 24 in einer Durchbrechung 38 des Sperrschiebers 18 angeordnet. Die Durchbrechung 38 ist zwischen dem Betätigungsbereich 20 und dem Kanal 50 angeordnet. Das erste, dem Betätigungsbereich 20 des Sperrschiebers 18 abgewandte Ende des Federelements 24 ist an einer Stützfläche 56 im Bereich des Kanals 50 abgestützt. Das zweite, dem Betätigungsbereich 20 zugewandte Ende des Federelements 24 ist an der Stoßfläche 58 des Sperrschiebers 18 abgestützt. Die Stützfläche 56 kann wie dargestellt an einem hülsenartigen Bauteil 26 vorgesehen sein.

Zur Durchführung des Betätigungselementes 42 ist eine Öffnung 40 in den Sperrschieber 18 eingearbeitet. Die Öffnung 40 fluchtet mit dem axialen Kanal 50, der sich vom Schaftrohr 12 bis zum Endkörper 16 durch das Instrument 10 erstreckt. Das als Zug-/Druckstange ausgeführte Betätigungselement 42 ist axial durch das Schaftrohr 12, den Verbindungskörper 14 und den Endkörper 16 geführt. Innerhalb des Verbindungskörpers 14 ist das Betätigungselement 42 durch das hülsenartige Bauteil 26 geführt.

In der Darstellung der Fig. 1 ist das medizinische Instrument 10 mit einem von dem Verbindungskörper 14 abgekuppelten Endkörper 16 gezeigt. Zur Herstellung einer Kupplungsverbindung zwischen dem Verbindungskörper 14 und dem Endkörper 16 wird der Endkörper 16 in der dargestellten Ausführungsform in eine axial Aufnahmebohrung des Verbindungskörpers 14 gesteckt, derart dass der Rasthaken 44 des Sperrschiebers 18 in eine Rastnut des Endkörpers 16 greift. Wie dargestellt, kann der Rasthaken 44 eine schräge Anlauffläche aufweisen, die beim Einstecken des Endkörpers 16 in den Verbindungskörper 14 gegen einen Randbereich der Rastnut stößt und den Sperrschieber 18 zwangsgeführt gegen die durch das Federelement 24 aufgebrachte Federkraft in dem Schacht 22 verschiebt. Bei der weiteren Verschiebung des Endkörpers 16 in den Verbindungskörper 14 rastet der Rasthaken 44 in die Rastnut des Endkörpers 16 und verriegelt diesen formschlüssig an dem Verbindungskörper 14. Wie dargestellt, kann vorgesehen sein, dass der Endkörper 16 drehbar an dem Verbindungskörper 14 gehalten ist. Dafür ist die Rastnut des Endkörpers 16 kreisringförmig ausgeführt.

An dem Endkörper 16 ist ein elektrischer Anschluss 32 vorgesehen, über den ein Signalgenerator wie zum Beispiel ein Hochfrequenzgenerator, eine Stromquelle und/oder eine Spannungsquelle an das medizinische Instrument 10 anschließbar ist. Bei der Ausführung des medizinischen Instruments 10 als bipolares Instrument, können an dem elektrischen Anschluss 32 mehrere Kontakte vorgesehen sein, über die elektrische Signale bzw. elektrische Spannungen getrennt voneinander an das medizinische Instrument 10 weiterleitbar sind. Über eine elektrische Verbindung 36 kann eine elektrische Spannung von dem elektrischen Anschluss 32 an einen insbesondere als Schleifkontakt ausgebildeten Kontakt 34 weitergeleitet werden. Über den Kontakt 34 kann die elektrische Spannung über das Bauteil 26 innerhalb des Verbindungskörpers 14 an das Schaftrohr 12 weitergeleitet werden. Das Bauteil 26 kann dafür einen elektrisch leitfähigen Pfad aufweisen oder aus einem elektrisch leitfähigen Material hergestellt sein. Es kann vorgesehen sein, dass der Kontakt 34 beim Einkuppeln bzw. in der Kupplungsstellung des Endkörpers 16 an dem Verbindungskörper 14 einen elektrisch leitenden Kontakt mit einer Oberfläche des Bauteils 26 herstellt. Das Bauteil 26 kann dafür zum Beispiel zumindest an einem Bereich der Oberfläche aus einem Metall wie Edelstahl hergestellt sein. Bevorzugt ist das Bauteil 26 aus einem elektrisch leitfähigen Vollmaterial hergestellt.

Zur Weiterleitung an das Schaftrohr 12 kann das Bauteil 26 die elektrische Spannung an einen Kontakt 48 des Schaftrohres 12 übergeben. Von dem Kontakt 48 kann das elektrische Signal über einen elektrisch leitfähigen Pfad 36' an den distalen Endbereich des Schaftrohres 12 weitergeleitet und beispielsweise an einen Endeffektor (nicht dargestellt) abgegeben werden.

Wie aus Fig. 1 ersichtlich, ist das als Schraubenfeder ausgebildete Federelement 24 an seinem unteren Ende gegen das elektrisch leitfähige Bauteil 26 innerhalb des Verbindungskörpers 14 abgestützt. Bei einer Ausführung des Bauteils 26 aus einem elektrisch leitfähigen Vollmaterial und der Ausführung des Federelements 24 ebenfalls aus einem elektrisch leitfähigen Material wie einem Edelstahl, ist die Feder 24 ebenfalls mit der durch das Bauteil 26 geführten Spannung beaufschlagt.

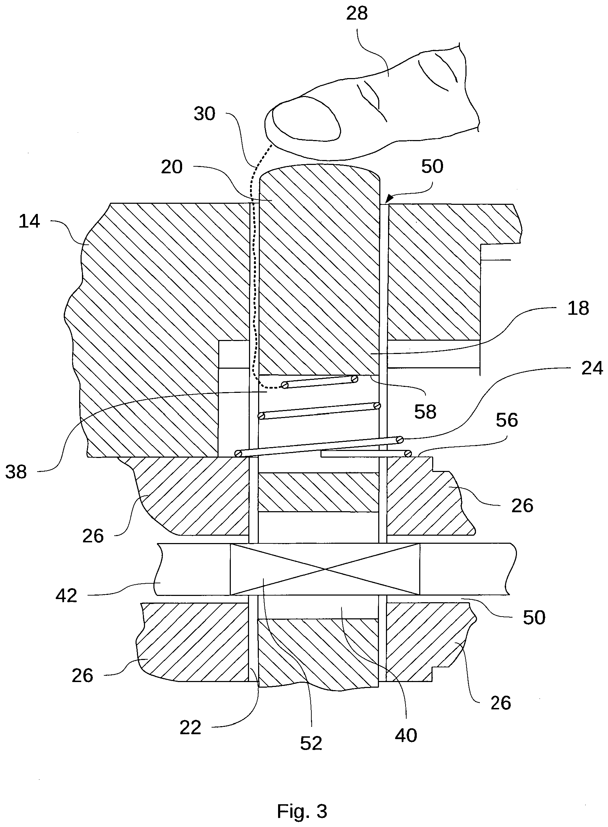

Wie in Fig. 3 gezeigt, kann die Gefahr bestehen, dass eine elektrische Spannung an der Schraubenfeder 24 über einen Spalt 50 an einen Bediener, insbesondere an einen Finger 28 eines Bedieners des medizinischen Instruments 10 überschlägt. Rein schematisch ist in Fig. 3 bei 30 eine Funkenstrecke bzw. Kriechstromstrecke gezeigt, die zwischen der Schraubenfeder 24 und einem Finger 28 eines Bedieners verläuft. Wie in Fig. 3 angedeutet, kann diese Strecke 30 dadurch verlängert werden, dass das Federelement 24 als Konusfeder ausgebildet ist. Bei der Verwendung einer graden Schraubenfeder ist die Strecke 30 kürzer, da eine grade ausgeführte Schraubenfeder 24 unmittelbar an den den Sperrschieber 18 umlaufenden Spalt 50 angrenzt. Mit einer konisch geformten Feder lassen sich also Vorteile hinsichtlich der Konstruktion und der elektrischen Sicherheit gegen Funkenüberschlag erzielen. Das breite Ende einer konisch geformten Feder kann wie in Fig. 3 dargestellt über die Ränder eines Durchbruchs 38 des Sperrschiebers 18 hinausragen und an einer Stützfläche 56 des Bauteils 26 abgestützt sein und an dem verjüngten Ende an einer Stoßfläche 58 unterhalb des Betätigungsbereiches 20 des Sperrschiebers 18 anliegen. Bei der Verschiebung des Sperrschiebers 18 in dem Schacht 22 können sich keine am oberen Ende der Feder 24 über den Öffnungsrand der Durchbrechung 38 hinausragenden Bereiche in dem Schacht 22 verklemmen.

Fig. 2 zeigt den Sperrschieber 18 und den Verbindungskörper 14 geschnitten entlang der Linie 2-2 aus Fig. 1. Über die radial nach außen abstehenden Griffflächen 46 an dem Verbindungskörper 14 kann ein Bediener den Verbindungskörper 14 zusammen mit dem Schaftrohr 12 gegenüber einem an dem Endkörper 16 gelagerten Handgriff (vgl. Fig. 4) verdreht werden. Dafür ist der Endkörper 16 drehbar an den Verbindungköper 14 ankuppelbar. Wie in Fig. 2 dargestellt, ist der Raum unterhalb des Sperrschiebers 18 innerhalb des Verbindungskörpers 14 verrundet ausgebildet. Damit kann die Wandstärke des Verbindungskörpers 14 im Bodenbereich des Schachtes 20 dünn ausgebildet sein, ohne die Bruchgefahr in diesem Bereich zu erhöhen. Geringe Wandstärken sind wünschenswert, um schmale und leichte Instrumente bereitzustellen.

Fig. 3 zeigt einen Detailausschnitt des Verbindungskörpers 14 aus Fig. 1 in einer schematischen Schnittdarstellung, nämlich insbesondere einen Bereich des Schachtes 22, in dem der Sperrschieber 18 läuft. In dieser Darstellung ist erkennbar, dass der Sperrschieber 18 per Fingerdruck über den Finger 28 eines Benutzers des medizinischen Instruments teilweise in den Schacht 22 eingedrückt ist. Dabei ist das als konische Schraubenfeder ausgebildete Federelement 24 leicht zusammengedrückt. Das Federelement 24 stützt sich an seinem unteren Ende gegen die Stützfläche 56 oberhalb des Kanals 50 ab. An seinem oberen Ende ist das Federelement 24 an der Stoßfläche 58 des Sperrschiebers 18 abgestützt. Unterhalb des Federelements 24 ist die Öffnung 40 angedeutet, die einen Eingriffsbereich 52 des Betätigungselements 42 umgreift. Die Stützfläche 56 ist an der Oberfläche des Bauelements 26 ausgebildet.

In Fig. 3 ist zur Verdeutlichung übertrieben dargestellt ein Spalt 50 erkennbar, der zwischen der Innenwandung des Schachtes 22 und der Außenwandung des Sperrschiebers 18 verläuft. Die gestrichelt dargestellte Linie 30 markiert eine potentielle Funkenstrecke bzw. Kriechstromstrecke zwischen dem Federelement 24 und dem Finger 28 eines Benutzers des Instruments 10. Wie deutlich in Fig. 3 dargestellt, verläuft die Kriechstromstrecke 30 durch den Spalt 50 um eine Kante des Sperrschiebers 18 bis an das obere Ende des als konische Schraubenfeder ausgebildeten Federelements 24. Durch die Verwendung einer konischen Schraubenfeder wird die elektrische Sicherheit des medizinischen Instruments 10 mindestens in zweierlei Hinsicht erhöht. Einerseits wäre die Kriechstromstrecke 30 bei der Verwendung einer graden Schraubenfeder kürzer, da das obere Ende der Schraubenfeder unmittelbar im Bereich des Spaltes endet und andererseits wird ein direkter Weg innerhalb des Spalts 50 verhindert, da das obere Ende des Federelements 24 mit einem gegenüber dem Sperrschieber 18 kleinen Querschnitt hinter die äußeren Grenzen nach innen eingerückt ist. Die Kriechstromstrecke 30 ist somit um eine Kante im Bereich der Stoßfläche 58 des Sperrschiebers 18 geführt. Diese konstruktive Ausgestaltung verbessert die elektrische Sicherheit des medizinischen Instruments 10 dahingehend, dass die Gefahr einer Spannungsübertragung von dem Federelement 24 zu dem Finger 28 eines Bedieners durch den Spalt 50 verringert wird.

Fig. 4 zeigt den Endkörper 16 aus Fig. 1 mit einem daran befestigten Handgriff 54. Erkennbar ist, dass der Handgriff 54 an seinem oberen Ende mit dem Betätigungselement 42 bewegungsgekoppelt ist. Mit der Betätigung des Handgriffs 54 kann eine Zug- bzw. Druckkraft auf das Betätigungselement 42 übertragen werden. Der Handgriff 54 ist vorzugsweise integral an den Endkörper 16 angeformt. Alternativ kann eine lösbare Verbindung zwischen Handgriff 54 und Endkörper 16 vorgesehen sein.

### Bezugszeichenliste

- 10: medizinisches Instrument
- 12: Schaftrohr
- 14: Verbindungskörper
- 16: Endkörper
- 18: Sperrschieber
- 20: Betätigungsbereich
- 22: Schacht
- 24: Federelement
- 26: Bauteil mit Stützfläche
- 28: Finger
- 30: Kriechstrom- / Funkenstrecke
- 32: elektrischer Anschluss
- 34: elektrischer Kontakt
- 36, 36': elektrischer Pfad
- 38: Durchbrechung im Sperrschieber
- 40: Öffnung im Sperrschieber
- 42: Betätigungselement
- 44: Rasthaken
- 46: Grifffläche
- 48: elektrischer Kontakt
- 50: Spalt
- 52: Eingriffsbereich
- 54: Handgriff
- 56: untere Stützfläche für das Federelement
- 58: obere Stoßfläche für das Federelement

## Patentansprüche

1. Medizinisches Instrument (10) für endoskopische Chirurgie, aufweisend ein Schaftrohr (12) mit einem distalen und einem proximalen Ende und einen im Bereich des proximalen Endes des Schaftrohres (12) angeordneten Verbindungskörper (14), wobei das Schaftrohr (12) und der Verbindungskörper (14) zur Durchführung eines langgestreckten Betätigungselements (42) axial von einem gemeinsamen Kanal (50) durchsetzt sind, einen an den Verbindungskörper (14) ankuppelbaren Endkörper (16) mit einem daran befestigten Handgriff (54) zur Bedienung des medizinischen Instrumentes (10), einen Sperrschieber (18), der in einem im Winkel zu dem Kanal (50) ausgerichteten Schacht (22) innerhalb des Verbindungskörpers (14) verschiebbar gelagert ist, wobei der Sperrschieber (18) mit einem Betätigungsbereich (20) aus dem Verbindungskörper (14) herausragt und per Fingerdruck auf den Betätigungsbereich (20) in eine Verriegelungs- und eine Freigabestellung betätigbar ist, derart, dass der an den Verbindungskörper (14) angekuppelte Endkörper (16) in der Verriegelungsstellung formschlüssig an dem Verbindungskörper (14) verriegelt ist und in der Freigabestellung von dem Verbindungskörper (14) abnehmbar ist, und wobei der Sperrschieber (18) mit Federkraft eines Federelements (24) in der Verriegelungsstellung gehalten ist, **dadurch gekennzeichnet, dass** das Federelement (24) in dem Schacht (22) zwischen dem Betätigungsbereich (20) und dem Kanal (50) angeordnet ist, und wobei das Federelement (24) als eine konisch geformte Schraubenfeder ausgebildet ist, dessen verjüngtes Ende an einer Stoßfläche (58) unterhalb des Betätigungsbereichs (20) des Sperrschiebers (18) abgestützt ist und dessen breites Ende auf einer Stützfläche (56) im Bereich des Kanals (20) abgestützt ist.

2. Medizinisches Instrument nach Anspruch 1, **dadurch gekennzeichnet, dass** das Federelement (24) in einer Durchbrechung (38) des Sperrschiebers (18) angeordnet ist.

3. Medizinisches Instrument nach einem der Ansprüche 1 oder 2, **dadurch gekennzeichnet, dass** an dem Endkörper (16) eine Anschlussstelle (32) angeordnet ist, zur Einkopplung einer elektrischen Spannung in das Schaftrohr (12) und/oder in ein durch den Kanal (50) geführtes Betätigungselement (42).

4. Medizinisches Instrument nach einem der vorangegangenen Ansprüche, **dadurch gekennzeichnet, dass** der Sperrschieber (18) eine das Betätigungselement (42) durchlassende Öffnung (40) aufweist.

5. Medizinisches Instrument nach Anspruch 4, **dadurch gekennzeichnet, dass** die Öffnung (40) einen Durchlassbereich und einen Fassbereich aufweist, wobei der Sperrschieber (18) in der Freigabestellung einen proximalen Abschnitt eines durch den Kanal (50) geführten Betätigungselements (42) durch den Durchlassbereich frei durchlässt und in der Verriegelungsstellung einen Eingriffsbereich (52) des Betätigungselements (42) drehformschlüssig mit dem Fassbereich umgreift.

6. Medizinisches Instrument nach einem der vorangegangenen Ansprüche, **dadurch gekennzeichnet, dass** der Sperrschieber (18) zumindest in dem für das Federelement (24) vorgesehenen Abschnitt zwischen dem Betätigungsbereich (20) und dem Kanal (50) einen Querschnitt aufweist, der in einer ersten Richtung schmaler und in einer zweiten Richtung breiter ist, als ein Querschnitt am ersten Ende des Federelements (24).

7. Medizinisches Instrument nach einem der vorangegangenen Ansprüche, **dadurch gekennzeichnet, dass** der Endkörper (16) in seiner an den Verbindungskörper (14) angekuppelten Gebrauchsstellung drehbar an dem Verbindungskörper (14) gelagert ist.

## Claims

1. A medical instrument (10) for endoscopic surgery, featuring a shaft tube (12) comprising a distal and a proximal end, and a connecting body (14) arranged in the vicinity of the proximal end of the shaft tube (12), wherein the shaft tube (12) and the connecting body (14) are axially penetrated by a common channel (50) for passing a longitudinal actuating element (42) therethrough, an end body (16) that can be coupled to the connecting body (14) and comprises a handle (54) attached thereto for operating the medical instrument (10), a locking slider (18) which is mounted displaceably in a shaft (22) oriented at an angle to the channel (50) inside the connecting body (14), wherein the locking slider (18) protrudes from the connecting body (14) with an actuating region (20) and can be actuated into a locking position and a release position at the touch of a finger on the actuating region, such that, in the locking position, the end body (16) coupled to the connecting body (14) is locked on the connecting body (14) in a form-fitting manner and, in the release position, can be removed from the connecting body (14), and wherein the locking slider (18) is kept in the locking position by means of the spring force of a spring element (24),
**characterised in that** the spring element (24) is arranged in the shaft (22) between the actuating region (20) and the channel (50), and wherein the spring element (24) is formed as a conically shaped helical spring, the tapered end of which is supported against an abutting surface (58) below the actuation region (20) of the locking slider (18) and the wide end of which is supported against a supporting surface (56) in the vicinity of the channel (50).

2. The medical instrument in accordance with Claim 1, **characterised in that** the spring element (24) is arranged in a through hole (38) of the locking slider (18).

3. The medical instrument in accordance with any one of Claims 1 or 2, **characterised in that** a connection point (32) is arranged at the end body (16) for injecting an electrical voltage into the shaft tube (12) and/or into an actuating element (42) passed through the channel (50).

4. The medical instrument in accordance with any one of the preceding claims,
**characterised in that** the locking slider (18) includes an opening (40) letting the actuating element (42) pass therethrough.

5. The medical instrument in accordance with Claim 4, **characterised in that** the opening (40) includes a passage region and a grasping region wherein, in the release position, the locking slider (18) lets a proximal section of an actuating element (42) passed through the channel (50) freely pass through the passage region and, in the locking position, grasps around an engagement region (52) of the actuating element (42) with the grasping region in a rotary form-fitting manner.

6. The medical instrument in accordance with any one of the preceding claims,
**characterised in that,** at least in the section provided for the spring element (24) between the actuating region (20) and the channel (50), the locking slider (18) includes a cross-section that is smaller than a cross-section at the first end of the spring element (24) in a first direction and wider than a cross-section at the first end of the spring element (24) in a second direction.

7. The medical instrument in accordance with any one of the preceding claims,
**characterised in that** the end body (16) is rotatably supported against the connecting body (14) when it is coupled to the connecting body (14) in its position of use.

## Revendications

1. Instrument médical (10) pour la chirurgie endoscopique présentant une gaine (12) avec une extrémité distale, une extrémité proximale et un corps de jonction (14) agencé au niveau de l'extrémité proximale de la gaine (12), la gaine (12) et le corps de jonction (14) étant traversés axialement par un canal commun (50) permettant le passage d'un élément d'actionnement (42) allongé, un corps d'extrémité (16) pouvant être accouplé au corps de jonction (14) et doté d'une poignée (54) qui y est fixée pour actionner l'instrument médical (10), un coulisseau de verrouillage (18) logé de façon à pouvoir y être déplacé dans une cavité (22) à l'intérieur du corps de jonction (14), cette cavité formant un angle par rapport au canal (50), le coulisseau de verrouillage (18) faisant saillie hors du corps de jonction (14) avec une partie d'actionnement (20) et pouvant, par une pression du doigt sur cette partie d'actionnement (20), être placé en une position de verrouillage ou une position de libération de façon à ce qu'en position de verrouillage le corps d'extrémité (16) accouplé au corps de jonction (14) soit bloqué contre celui-ci par complémentarité de formes et qu'il puisse, en position de libération, être détaché du corps de jonction (14), le coulisseau de verrouillage (18) étant maintenu en position de verrouillage par la force de ressort d'un élément ressort (24), **caractérisé en ce que** l'élément ressort (24) est agencé dans la cavité (22) entre la partie d'actionnement (20) et le canal (50), l'élément ressort (24) étant façonné en forme de ressort à boudin conique dont l'extrémité de section décroissante s'appuie contre une surface de butée (58) sous la partie d'actionnement (20) du coulisseau de verrouillage (18), l'extrémité large s'appuyant contre une surface d'appui (56) au niveau du canal (20).

2. Instrument médical selon la revendication 1, **caractérisée en ce que** l'élément ressort (24) est placé dans une percée (38) du coulisseau de verrouillage (18).

3. Instrument médical selon l'une des revendications 1 ou 2, **caractérisé en ce qu'**une borne de raccordement (32) est placée sur le corps d'extrémité (16) pour le branchement d'une tension électrique sur la gaine (12) et/ou un élément d'actionnement (42) introduit par le canal (50).

4. Instrument médical selon l'une des revendications précédentes, **caractérisé en ce que** le coulisseau de verrouillage (18) présente une ouverture (40) permettant le passage de l'élément d'actionnement (42).

5. Instrument médical selon la revendication 4, **caractérisée en ce que** l'ouverture (40) présente une zone de passage et une zone de serrage, le coulisseau de verrouillage (18) permettant, en position de libération, le passage libre d'une section proximale d'un élément d'actionnement (42) introduit à travers le canal (50) et enserrant, en position de verrouillage, avec la zone de serrage une zone d'engagement (52) de l'élément d'actionnement (42) maintenue par engagement positif s'opposant à la rotation libre.

6. Instrument médical selon l'une des revendications précédentes, **caractérisé en ce que** le coulisseau de verrouillage (18) présente, au moins dans la partie prévue pour l'élément ressort (24) entre la partie d'actionnement (20) et le canal (50), une section qui, dans une première direction est plus étroite et dans une seconde direction plus large qu'une section de la première extrémité de l'élément ressort (24).

7. Instrument médical selon l'une des revendications précédentes, **caractérisé en ce que** le corps d'extrémité (16), en sa position d'utilisation accouplée au corps de jonction (14), est logé dans le corps de jonction (14) de façon à pouvoir tourner.
